# EUROPEAN PATENT SPECIFICATION

(11) **EP 2 935 604 B1**
(45) Date of publication and mention of the grant of the patent: **14.11.2018**
(21) Application number: 13818498.1
(22) Date of filing: 18.12.2013
(51) Int. Cl.: C12P 19/02, C12P 19/14

(54) **PROCESS FOR ENZYMATIC HYDROLYSIS OF CELLULOSE WITH PROTEIN ADDITION**
VERFAHREN ZUR ENZYMATISCHEN HYDROLYSE VON CELLULOSE MIT PROTEINZUGABE
PROCÉDÉ D'HYDROLYSE ENZYMATIQUE DE CELLULOSE AVEC L´ADDITION D'UNE PROTEIN

(30) Priority: 18.12.2012 NL 2010006
(43) Date of publication of application: 28.10.2015
(73) Proprietor: Stichting Energieonderzoek Centrum Nederland, 1755 LE Petten (NL)
(72) Inventor: SMIT, Adrianus Theodorus, NL-1764 KM Breezand (NL); HUIJGEN, Wouter Johannes Joseph, NL-1814 HV Alkmaar (NL)
(74) Representative: Nederlandsch Octrooibureau
(86) International application number: PCT/NL2013/050918
(87) International publication number: WO 2014/098589

(56) References cited:
- HAN ET AL: "Synergism between hydrophobic proteins of corn stover and cellulase in lignocellulose hydrolysis", BIOCHEMICAL ENGINEERING JOURNAL, vol. 48, 2010, pages 218-224, XP026809946, cited in the application
- BALS ET AL: "Economic comparison of multiple techniques for recovering leaf protein in biomass processing", BIOTECHNOLOGY AND BIOENGINEERING, vol. 108, 2011, pages 530-537, XP055054727, cited in the application
- WILDSCHUT ET AL: "Ethanol-based organosolv fractionation of wheat straw for the production of lignin and enzymatically digestible cellulose", BIORESOURCE TECHNOLOGY, vol. 135, 23 October 2012 (2012-10-23), pages 58-66, XP002712539, cited in the application
- CHIESA ET AL: "Protein extraction from biomass in a bioethanol refinery - possible dietary applications: Use as animal feed and potential extension to human consumption", BIORESOURCE TECHNOLOGY, vol. 102, 2011, pages 427-436, XP028371434, cited in the application
- HUIJGEN ET AL: "Fractionation of wheat straw by prehydrolysis, organosolv delignification and enzymatic hydrolysis for production of sugars and lignin", BIORESOURCE TECHNOLOGY, vol. 114, 7 March 2012 (2012-03-07), pages 389-398, XP028422595,
- SU ET AL: "Supplementing with non-glycoside hydrolase proteins enhances enzymatic deconstruction of plant biomass", PLOS, vol. 7, August 2012 (2012-08), pages 1-11, XP002720439,
- RAHIKAINEN ET AL: "Inhibitory effect of lignin during cellulose bioconversion: The effect of lignin chemistry on non-productive enzyme adsorption", BIORESOURCE TECHNOLOGY, vol. 133, 26 January 2013 (2013-01-26), pages 270-278, XP002712589,
- KIM ET AL: "Front-end recovery of protein from lignocellulosic biomass and its effect on chemical pretreatment and enzymatic saccharification", BIOPROCESS AND BIOSYSTEMS ENGINEERING, vol. 36, 2 February 2013 (2013-02-02), pages 687-694, XP002720440,

## Description

The present invention is in the field of utilisation of biomass and is directed to an advanced process for the enzymatic hydrolysis of cellulose.

### Background

Biomass, especially lignocellulosic biomass, is a valuable resource for the production of (bio)fuels, chemicals, performance products and energy. Lignocellulose is the most abundant renewable biomass available on land, and therefore relatively cheap. It comprises mainly cellulose, hemicellulose and lignin. Many research efforts have been devoted to the development of processes for the cost-effective conversion of biomass, especially lignocellulose biomass, to valuable compounds. An example thereof is the conversion of cellulose to glucose, which in turn may serve as a precursor for 'second-generation' bioethanol (by fermentation of glucose), and is thus suitable for the preparation of biofuels.

Enzymatic hydrolysis of cellulose to glucose is still not applied on a commercial scale, since it cannot compete yet with glucose produced from first generation biomass sources (starch, sucrose etc.), among others, in view of the high costs of the required amounts of cellulolytic enzyme. Alternatives to enzymatic hydrolysis of cellulose, e.g. concentrated acid treatment, are undesirable for environmental reasons and because they yield more by-products by sugar degradation reactions. Hence, one of the challenges of current research is to find means to reduce the cost of enzymatic hydrolysis of cellulose, in order to allow industrial application, for example by enhancing enzymatic activity and reducing the required enzyme dose.

Pretreatment of biomass is a process in which the cellulose polymers are made better accessible for hydrolytic enzymes. Without pre-treatment, the cellulose within lignocellulosic biomass is poorly accessible for the hydrolytic enzymes, among others, as it is shielded by lignin and hemicelluloses. Pretreatment breaks down (and often partly removes) this shield of lignin and hemicellulose, and disrupts the crystalline structure of the cellulose and may even reduce the degree of polymerisation of the cellulose. Pretreatment may involve disrupting the cellular structure of the biomass and may remove lignin and/or hemicellulose from the biomass, depending on the type of pretreatment. Different types of pretreatment are known in the art (e.g. from Zheng et al., Int. J. Agric. & Biol. Eng. 2009, 2, 51-68. These types can in general be classified as chemical or mechanical pretreatment. Chemical pretreatment includes catalysed steam explosion, acid pretreatment, alkaline pretreatment, ammonia fibre/freeze explosion, organosolv, aquathermolysis, pH-controlled liquid hot water pretreatment and ionic liquid pretreatment. Physical pretreatment includes non-catalysed steam explosion, mechanical comminution and high energy radiation.

The cellulose-containing product (sometimes called pulp) obtained by the pre-treatment often comprises significant amounts of non-cellulose components. Even if the pretreatment involves removal of (large parts of) hemicellulose and lignin, these components are still present in the cellulose-containing product to some extent. Treatment with a water-miscible organic solvent at elevated temperature, known as "organosolv" treatment, for example, separates lignin and hemicellulose from the cellulose pulp (see: Wildschut et al. Bioresource Technology, 135 (2013), 58-66). Nevertheless, this cellulosic product comprises approximately 10 to 30 wt% of the lignin, based on the total original lignin content of the biomass. This fraction of the lignin present in lignocellulose biomass is highly resistant towards delignification. The impurities in the cellulose-rich pulp can hinder access of the enzyme to the cellulose during enzymatic hydrolysis. Also, the hydrolytic enzyme will irreversibly adsorb onto the lignin, whereby the enzyme is deactivated. Non prior art document Rahikainen et al., Biores. Technol., 2013, 133, 270-278 teaches lignin isolated from spruce and wheat straw pretreated by steam explosion has an inhibitory effect on hydrolysability.

It has been suggested to use commercial purified proteins in the enzymatic hydrolysis of cellulose so as to counteract the inhibiting effect of the biomass by-products, such as corn stover proteins (Han and Chen, Biochemical Engineering Journal, 48 (2010) 218-224), bovine serum albumin (Yang and Wyman, Biotechnology and Bioengineering, 94 (2006), 611-617, or Pan et al., Applied Biochemistry and Biotechnolgy, 121-124 (2005), 1069-1079). This still does not result in a commercially feasible process, because of the cost of the commercial purified proteins. Extracting proteins from biomass has been described by Bals and Dale (Biotechnology and Bioengineering, 198 (2011), 530-537) and by Chiesa and Gnansounou (Bioresource Technology, 102 (2011), 427-436). Non prior art knowledge on the role of protein removal on enzyme cellulose digestibility can be found in Kim et al., Bioprocess Biosyst. Eng. 2013, 36(6):687-94.

To date, no commercially feasible method to counteract the deactivating effect of residual lignin is available. The present invention meets the need, which exists in the art, for such a method.

### Summary of the invention

Surprisingly, the inventors have found that enzyme activity during enzymatic hydrolysis of cellulose is significantly enhanced when an aqueous extract, in particular a protein-containing extract, from biomass is added during enzymatic hydrolysis. As such, the enzyme loading during enzymatic hydrolysis step can be significantly reduced, without negatively affecting the yield of glucose. Surprisingly, the protein-rich aqueous biomass extract, suitable for enhancing the activity of the hydrolysing enzyme, may efficiently be prepared by washing the biomass with water and subsequently filtering the mixture. No further purification steps are necessary for achieving a significant raise in enzyme activity during enzymatic hydrolysis. Other components, which may be co-extracted from the biomass during aqueous extraction, do not inhibit the hydrolytic enzyme (cellulase) during enzymatic hydrolysis of cellulose, or this inhibition is more than compensated by the activity raise caused by the process according to the invention. Protein from the biomass itself constitutes a relatively cheap and easily accessible protein source. As a result of the reduced enzyme requirement, this invention meets the need for reducing the costs of enzymatic hydrolysis of cellulose.

Without being bound to theory, the inventors assume that proteins from the aqueous extract are adsorbed to the lignin present in the cellulosic substrate. As such, the amount of hydrolytic enzyme inactivated by adsorption onto liberated lignin decreases.

### Detailed description

The invention pertains to a process for enzymatic hydrolysis of cellulose contained in biomass, comprising:
(a) extracting biomass containing both protein and cellulose with an aqueous liquid to produce an extracted biomass and an aqueous extract having a protein content of at least 0.01 g per litre water;
(b) mechanically, chemically and/or thermally pretreating cellulose-containing-biomass; and
(c) contacting biomass pretreated in step (b) with:
   (i) an enzyme capable of hydrolysing cellulose; and
   (ii) the aqueous extract obtained in step (a).
The aqueous extract contains at least a part of the protein from the biomass, and is referred to below as protein-containing extract.

Biomass suitable for the process according to the invention includes lignocellulosic biomass, such as forestry residues, agricultural residues, yard waste, animal and human waste. Such biomass comprises in general 40 to 80 wt.% carbohydrates (based on dry matter), which are valuable starting materials for biorefinery. The cellulose-containing biomass used preferably also contains significant amounts of proteins; if not, e.g. in the case of wood biomass, the biomass used for extracting protein is different from the biomass used for hydrolysing cellulose. Preferably, herbaceous biomass is used in the process according to the invention. Most preferably, the herbaceous biomass is in the form of a (agricultural) residue such as straw and grass.

In a preferred embodiment, the extracted biomass obtained in step (a) of the process is used as the cellulose-containing-biomass in step (b).

### Pre-extraction

In a preferred embodiment, the process according to the invention involves a step of pre-extraction prior to pretreatment. Pre-extraction according to the invention involves extracting (water-soluble) proteins from the biomass, prior to any heat treatment of said biomass at a temperature of at least 75 °C as further described below. The biomass subjected to the pre-extraction step may be fresh or dried biomass, optionally after removal of large impurities such as stones and pieces of metal, and optionally after chopping or milling to pieces for ease of handling (e.g. pieces of 0.01 to 50 cm, in particular 0.1-10 cm in length or diameter, depending on the type of biomass).

Pre-extraction is performed using an aqueous extracting solvent, in particular water. The water may be demineralised, but demineralisation is generally unnecessary. The aqueous liquid may contain agents assisting in the dissolution of proteins, such as acids, bases, salts and surfactants. The pH may be from slightly alkaline to acidic, e.g. between 2 and 10, preferably between 4 and 8. If desired, minor amounts of an organic solvent, such as alcohols, polyols, ethers and the like, may be added to the extraction solvent. However, the level of these organic solvents is preferably kept low, e.g. below 20 wt%, more preferably below 10 wt%, as they may cause unwanted precipitation of some of the proteins and/or unwanted co-extraction of further components from the biomass. It was found that (non-demineralised) tap water or filtered, relatively clean surface water can be conveniently used, while demineralised water is also suitable, with or preferably without added organic solvents or other additives. Also for reasons of cost, the additional use of organic solvents and additives is preferably minimised.

Pre-extraction may be performed by any extraction technique known in the art. Conveniently, pre-extraction is performed by washing the biomass with the solvent, or by soaking the biomass in the solvent. In this embodiment, the biomass preferably soaks at least 1 minute in the solvent, more preferably between 5 minutes and 600 minutes, most preferably between 10 minutes and 120 minutes.

The extraction may also be performed stage-wise, in a counter-current mode. In such a staged mode, relatively clean (protein-depleted) water is used for a second or later stage of the extraction and the extract of the second or later stage is used as an extracting liquid for the preceding (or first) stage. In this way the residual amount of water-soluble proteins in the biomass is minimised while keeping the amount of extracting liquid and hence the liquid load in the enzymatic hydrolysis relatively low. Counter-current extraction allows a reduction in the total amount of extraction solvent.

The extraction can be performed with water having a temperature between melting temperature (0°C) and boiling temperature (100°C or higher if pressurised), while preferably the temperature is chosen such that degradation of proteins is minimised. Preferred extracting temperatures are from 10 to 75 °C, more preferred from 15 to 60°C, most preferred from 20 to 50°C. The amount of extracting liquid (e.g. water) is preferably 1-20 litre of liquid per kg of biomass. For single stage extraction, the preferred amount is 2-12, most preferred 3-10 1 of water per kg of biomass. For counter-current extraction, the preferred amount of extraction solvent (water) is between 1 and 6, especially between 1.5 and 4 1 water per kg biomass. The biomass weight is understood herein as the dry weight, without adherent water.

The mixture of biomass and solvent may then be filtered, using a filter having small enough pores to retain the chopped and washed or soaked biomass, and large enough pores to allow the proteins to pass. Typically, the pores of such a filter are between 10 µm and 10 mm in diameter, preferably between 100 µm and 1 mm. The permeate is collected as the protein-rich aqueous extract, and the retentate comprising biomass is used for further treatment.

In an especially preferred embodiment, the biomass which has been washed (pre-extracted) for extraction of proteins, is further pretreated and subjected to enzymatic hydrolysis as described here below. Alternatively, the pre-extraction of biomass to prepare the protein-rich aqueous extract is performed on a different batch than the batch used for pretreatment and enzymatic hydrolysis according to the invention. The different batch may originate from the same biomass or from another biomass, e.g. a biomass having a different protein content. In this instance, the pre-extracted biomass may be used for any desired purpose.

In a preferred embodiment, the same batch of biomass is subjected to aqueous pre-extraction as described above and pretreatment as described below, and the extracted biomass obtained in the aqueous pre-extraction is subjected prior to the pretreatment to an additional extraction step with an organic liquid, i.e. a liquid comprising at least 20 wt% organic solvent, preferably at least 50 wt%, even more preferably at least 70 wt%, more preferably at least 90 wt%, most preferably at least 99 wt%. Suitable organic solvents to be used in this optional additional extraction step include, but are not limited to, lower alcohols and diols, ethers, ketones, amides, lower alkanes and carboxylic acids. In the context of the present invention, "lower" means containing 1-6 carbon atoms (C₁-C₆), especially C₁-C₄. The organic solvent is preferably water-miscible or capable of dissolving at least 10 wt% of water.

Examples of suitable organic solvents for the optional additional extraction step include methanol, ethanol, propanol, isopropanol, butanol and its isomers, ethylene glycol, propylene glycol, methoxyethanol, dimethoxyethane, diethylene glycol, dioxane, acetone, methyl ethyl ketone, tetrahydrofuran, dimethyl formamide, dimethyl acetamide, N-methylpyrrolidone etc. Further polar (co)solvents can be used as well, although these are slightly less preferred, for example acetonitrile, formic acid, acetic acid, methyl acetate, ethyl acetate, non-apolar haloalkanes such as dichloromethane, and CO₂ (sc). Apolar solvents, such as hydrocarbons, e.g. pentane, cyclopentane, hexane, toluene or mixtures thereof, such as petroleum ether, can be also used as (co)solvents. In the context of the present disclosure, mixtures of miscible organic solvents are also encompassed in the term "organic solvent". Preferably, the organic solvent is selected from methanol, ethanol, propanol, butanol and acetone.

The aqueous phase or the aqueous permeate of the aqueous pre-extraction of biomass comprises water-soluble components originating from the biomass, such as water-soluble proteins. The protein-rich aqueous extract may also comprise other water soluble components from the biomass, such as salts, non-structural carbohydrates (pentose and hexose mono- and oligosaccharides) and any other water-soluble matter. The aqueous phase or the aqueous permeate may be used as such, without further purification, in the enzymatic hydrolysis of cellulose as described below, or may be further processed prior to being used in enzymatic hydrolysis. Such further processing may include nanofiltration over a membrane having a molecular weight cut-off between 500 Da and 3 kDa. When nanofiltration is employed, the retentate comprises proteins and is considered to be the protein-rich aqueous biomass extract in the context of the present invention. The permeate may be returned to the extraction step.

Typically, lignocellulosic biomass may contain 30 % of proteins (dry weight basis), depending on the type of biomass. However, the use of biomass having higher proteins levels is not excluded, and may even be advantageous. Grasses may contain e.g. 15-30 wt%, while most straws have between 2 and 8 wt% of proteins (on dry matter basis). During the aqueous pre-extraction at least 10 wt% of the water-soluble proteins is extracted from the biomass (typically from 20 to 100 wt%). The concentration of the protein in the aqueous extract is not essential for the application in enzymatic hydrolysis of cellulose according to the invention.

The protein content of the aqueous extract obtained in step (a) is at least 0.01, preferably at least 0.05, more preferably at least 0.1 g per litre water, up to the solubility limit, which may be between 10 and 50 g/l, depending on the particular proteins. In particular, the protein content is at least 0.2 g/l, more preferably at least 0.5 g/l, most preferably at least 1 g/l, e.g. up to 20 g or up to 15 g per litre water. The protein content of the extract as used in step (c) may have the same protein content. If the protein content is considered to be too low, the protein-rich aqueous extract may be concentrated or diluted prior to being used in the enzymatic cellulose hydrolysis according to the invention, for example by nanofiltration, to obtain a protein concentration of e.g. 0.2-30, in particular 1-20 g/l. In a preferred embodiment, no further processing steps are carried out on the protein-rich aqueous extract, other than filtration or concentration of the extract.

### Pretreatment

The process according to the invention may involve any type of mechanical, chemical and/or thermal treatment known in the art for pretreatment of lignocellulosic biomass for the purpose of enzymatic hydrolysis. The process is also sometimes referred to as cooking or pulping. Pretreatment of the biomass is necessary for making the cellulose accessible for enzymatic hydrolysis. Suitable mechanical and/or thermal treatment steps include, but are not limited to, catalysed steam explosion, acid pretreatment, alkaline pretreatment, ammonia fibre/freeze explosion, organosolv, pH-controlled liquid hot water pretreatment, uncatalysed steam explosion, aquathermolysis, liquid hot water pretreatment, mechanical comminution and high energy radiation.

Preferably, the pretreatment step is an organosolv step, i.e. pretreatment with a water-miscible solvent and possibly an (acidic) catalyst, a steam explosion step or an aquathermolysis step, most preferably an organosolv step. The water-miscible solvent for the organosolv step can be a lower alcohol, ether or ketone, such as methanol. ethanol, (iso)propanol, butanol, ethylene glycol, methoxyethanol, dimethoxyethane, dioxane, acetone, methyl ethyl ketone, etc. Preferred water-miscible solvents include ethanol and acetone. When applying the optional additional extraction step described above, it is preferred that the solvent to be used therein is the same solvent as used in the (organolsolv) pretreatment.

The pretreatment step is preferably performed at a temperature of at least 75 °C, more preferably at least 100 °C, up to 350 °C, preferably up to 280 °C, most preferably between 120 and 240°C. The pH can be between alkaline and strongly acidic, e.g. between 1 and 11, preferably between 1.5 and 7.0, more preferably between 2.0 and 5.0.

During organosolv treatment, parts of the lignin and hemicellulose are removed from the biomass. Thus, the resulting biomass is enriched in cellulose with a reduced lignin and hemicellulose content. During steam explosion and aquathermolysis, typically, hemicellulose is hydrolysed and lignin remains largely in the biomass. As a result, the content of lignin is typically still sufficiently high to markedly deactivate the hydrolytic enzyme during enzymatic hydrolysis.

Thus, it is preferred that step (b) of the process of the invention comprises treating with a water-miscible organic solvent, preferably selected from lower alcohols, lower ethers and lower ketones, optionally in the presence of an acid. Alternatively, or additionally, step (b) of the process of the invention comprises a steam explosion step or an aquathermolysis step.

### Enzymatic hydrolysis

Enzymatic hydrolysis of cellulose to glucose is accomplished by an enzyme or combination of enzymes capable of hydrolysing cellulose, referred to as cellulases. Hydrolysis of cellulose is also known as cellulolysis. The activity of cellulase enzymes is typically measured in FPU (filter paper unit); see Ghose, T. K. Measurement of cellulase activities. Pure Appl. Chem. 1987, 59, 257-268. The process according to the invention may be performed using any cellulase enzyme. Suitable cellulase enzymes are endocellulases (breaking cellulose at inner positions), exocellulases (cellobio-hydrolases, breaking cellulose at more external positions to produce cellobiose or cellutetraose), beta-glucosidases (cellobiases, cleaving the exocellulase products into glucose units). Other cellulose enzymes, such as oxidative cellulases and cellulose phosphorylases, are less preferred. Preferably a combination of cellulase enzymes is used, in particular a combination of endo-cellulase, exo-cellulase and β-glucosidase. Also, hemicellulases may advantageously be present to decompose any residual hemicellulose remaining after the pretreatment step. Hemicellulases which may be present primarily include xylanases, and may further includes xylosidases, arabinosidases, mannanases, galactanases etc.

In the process according to the invention, the enzymatic hydrolysis of cellulose may be performed in any manner known in the art. The process according to the invention is characterised by the presence of a protein-rich aqueous extract during enzymatic hydrolysis, wherein the protein-rich aqueous extract originates from biomass extraction. Thus, the cellulose-rich pulp or the pretreated biomass is contacted simultaneously with (i) an enzyme capable of hydrolysing cellulose, preferably a mixture of cellulases, and with (ii) a protein-rich aqueous extract originating from aqueous extraction of biomass. In an especially preferred embodiment, the protein-rich aqueous biomass extract used during the enzymatic hydrolysis of biomass originates from aqueous extraction of the same biomass, prior to pretreatment. The protein-rich extract may be the directly obtained extract or a concentrate thereof.

The protein-rich aqueous extract comprises native proteins. Thus, both the biomass, prior to aqueous extraction, and the protein-rich aqueous extract itself, prior to use thereof during enzymatic hydrolysis, preferably are not subjected to a heat treatment at a temperature above 100 °C, preferably not above 75 °C. Thus, it is especially preferred that the biomass used for preparing the protein-rich aqueous extract does not undergo pretreatment prior to aqueous extraction. The proteins which are present during enzymatic hydrolysis do not need to exhibit a certain activity during enzymatic hydrolysis. Their role is assumed to be their adsorption onto lignin, thus preventing the enzymes from being adsorbed to lignin. The concentration of the protein in the aqueous extract is not essential for the application in enzymatic hydrolysis of cellulose according to the invention. Typically, the higher the protein content, the higher the activity raise which is observed during enzymatic hydrolysis.

The amount of protein (contained in or originating from the aqueous extract) which is to be used during enzymatic hydrolysis may vary. In particular the amount can be proportional to the amount of pretreated biomass. It is preferred to use between 0.001 and 0.1 g of protein per g of pre-treated biomass (dry weight basis), more preferred between 0.02 and 0.05 g protein per g pre-treated biomass.

The treated biomass is brought in simultaneous contact with the hydrolytic enzyme and with the protein-rich aqueous extract, which means that both the hydrolytic enzyme and protein-rich aqueous extract should be present during at least part of the enzymatic hydrolysis. Thus, the hydrolytic enzyme may first be added to the pretreated biomass, or the protein-rich aqueous extract may first be added to the pretreated biomass, or the hydrolytic enzyme and the protein-rich aqueous extract are simultaneously brought in contact with the treated biomass. "Contacting" or "bringing in contact" indicates that each of the two or more components may be added to the other ones by any means known in the art. The method and order of addition are not critical for the beneficial effect on the activity of the hydrolytic enzyme. Also, as enzymatic hydrolysis typically takes one to four days, it is not essential whether the enzymatic hydrolysis has already commenced for some period of time before the protein-rich aqueous extract is added to the reaction mixture comprising pretreated biomass and hydrolytic enzyme. During enzymatic hydrolysis of cellulose, the polymeric structure of cellulose slowly decomposes, as such slowly liberating further lignin. Over time, more free lignin may become available in the reaction mixture, and thus more adsorption sites for proteins or the hydrolytic enzyme. Thus, it is important that for at least part of the enzymatic hydrolysis, the hydrolytic enzyme and the protein-rich aqueous extract are present simultaneously. Preferably, the protein-rich aqueous extract is present during at least 75 % of the enzymatic hydrolysis (i.e. 75 to 100 %), more preferably at least 90 %, most preferably at least 95 %.

The resulting hydrolysate is rich in glucose, which may be further processed, optionally after separation of solid residues, such as fermented to produce e.g. ethanol or other alcohols or chemically treated to produce e.g. 5-hydroxymethyl-furfural and other furans, or the glucose may be used as such, as known in the art.

### Examples

### Example 1: Organosolv and enzymatic hydrolysis for wheat straw:

Wheat straw was chopped to pieces of about 1 cm to 20 cm in size. The chopped wheat straw was washed (pre-extracted) with water (10 L per kg straw) at 50 °C for 60 minutes. The mixture was filtered over a 185 µm filter, resulting in an aqueous extract comprising protein ('protein-rich aqueous extract'). 100 mL of the protein-rich aqueous extract was filtered over a tangential flow membrane filtration system with a 1 kDa cut-off to remove the proteins from the extract, but not soluble components with a MW <1kDa such as salts. The resulting permeate is referred to as the protein-free extract.

Wheat straw of another batch (not subjected to water extraction) was chopped and subjected to organosolv conditions (OS) in a batch autoclave reactor. The OS conditions were: 190 °C, 60 minutes, solvent = ethanol/water (60/40% wt/wt, 10 L/kg dry matter), 30 mM H₂SO₄. OS gave a solid recovery (pulp yield) of 43.0 % (dry weight).

The remaining cellulose pulp after treatment of the biomass was subjected to enzymatic hydrolysis, using 5, 10, 15 or 20 FPU per gram pulp (substrate) of cellulase enzyme (Accellerase 1500, DuPont Industrial Biosciences). In the enzymatic hydrolysis, 1.50 g of pulp (originating from 3.49 g wheat straw) in a total volume of 50.0 ml (34.9 mL aqueous extract obtained from aqueous extraction of another batch of the same wheat straw + 15.1 mL water, buffered at pH 4.8, or 50 mL water, buffered at pH 4.8 (control)) were used. Different amounts of cellulase (5, 10, 15 or 20 FPU per gram pulp) were added and the progress of the enzymatic hydrolysis was monitored by determination of the glucose concentration at various intervals for 72 h. A cross-control experiment was performed, wherein the enzymatic hydrolysis was performed in 50.0 mL (34.9 mL protein-free extract + 15.1 mL water), in the presence of 10 FPU per gram pulp.

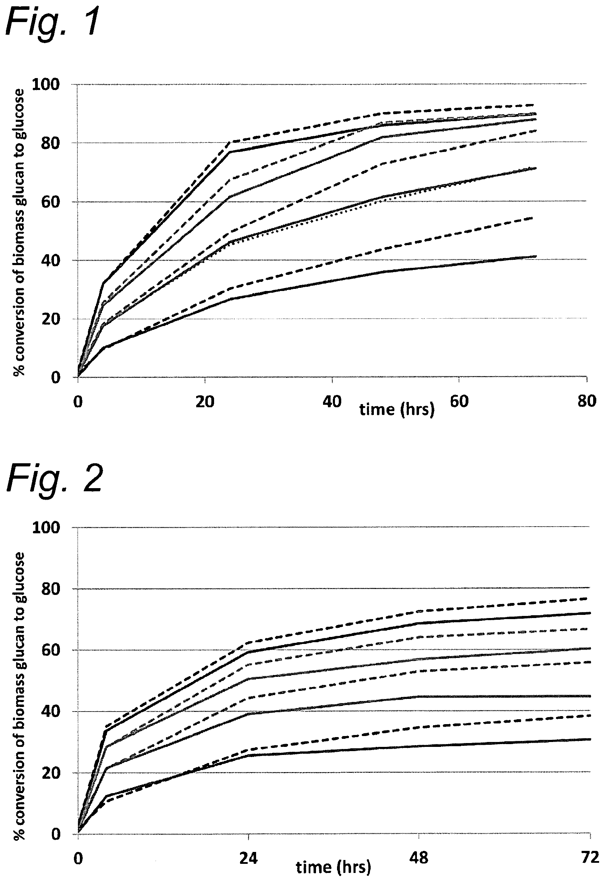

Figure 1 summarises the results obtained in Example 1 (enzymatic hydrolysis of wheat straw which underwent organosolv pretreatment). The solid lines represent the values obtained without protein extract (control experiments), the dashed lines represent the values obtained with the protein extract. The dotted line represents the 10 FPU/g values obtained with protein-free extract. The higher the enzyme concentrations (5-20 FPU/g), the higher the conversion percentage. Clearly, the dashed lines (enzymatic hydrolysis in water + extract) represent higher enzyme activity with the same enzyme loading than the solid lines (enzymatic hydrolysis in water). After 72 h, glucose yields were increased with approximately 30 % at the lowest enzyme loading (5 FPU/g). Also at higher enzyme loading increased yields were observed when protein-rich extract was present during enzymatic hydrolysis. When protein-free extract was used, no increase in glucose yield was observed.

### Example 2: Aquathermolysis for wheat straw:

Wheat straw was chopped and subjected to aquathermolysis conditions (AT) in a batch autoclave reactor. The AT conditions were: 190 °C, 60 minutes, solvent = water (15 L/kg dry matter). AT gave a dry weight yield of 59.9 %.

The remaining cellulose pulp after treatment of the biomass was subjected to enzymatic hydrolysis, using 5, 10, 15 or 20 FPU per gram pulp (substrate) of cellulase enzyme (Accellerase 1500, DuPont Industrial Biosciences). In the enzymatic hydrolysis 1.50 g of pulp (originating from 2.50 g wheat straw) in a total volume of 50.0 ml (25.0 mL protein-rich aqueous extract obtained from the another batch of wheat straw as described in Example 1, + 25.0 mL water (buffered at pH 4.8) or 50 mL water (control)). Different amounts of cellulase (5, 10, 15 or 20 FPU per gram pulp) were added and the progress of the enzymatic hydrolysis was monitored at various intervals for 72 h.

Figure 2 summarises the results obtained in Example 2 (enzymatic hydrolysis of wheat straw which underwent aquathermolysis). The solid lines represent the values obtained without protein extract (control experiments), the dashed lines represent the values obtained with the protein extract. The higher the enzyme concentrations (5-20 FPU/g), the higher the conversion degree. In view of the high lignin content of the resulting pulp, the hydrolytic enzyme was deactivated more rapidly, as the curves flattened rapidly after 24 h. Nevertheless, for all enzyme loadings, the resulting yield of glucose was increased when enzymatic hydrolysis was performed in water + extract, compared to enzymatic hydrolysis with the same enzyme loading in only water.

### Example 3: Organosolv for rice straw:

Rice straw was chopped and extracted as described in Example 1, and subjected to OS: 200 °C, 60 minutes, solvent = ethanol/water (60/40, 10 L/kg dry matter), 34 mM H₂SO₄. OS gave a solid recovery (pulp yield) of 43.4 %. The pulp obtained was subjected to enzymatic hydrolysis: 1.50 g of pulp (originating from 3.46 g rice straw) in a total volume of 50.0 ml (34.6 mL protein-rich aqueous extract obtained from the same rice straw + 15.4 mL water (pH 4.8) or 50 mL water (control)). Different amounts of cellulase (5, 10, 15 or 20 FPU per gram pulp) were added and the progress of the enzymatic hydrolysis was monitored at various intervals for 72 h. The results were comparable to those obtained for wheat straw (Example 1). The results using 34.6 mL protein-free extract + 15.4 mL water, in the presence of 10 FPU per gram pulp, were the same as the results obtained from the control (50 mL water) (not shown).

For all cellulose pulps tested, both with high lignin content (after aquathermolysis) or reduced lignin content (after organosolv), increased hydrolytic enzyme activity was observed when protein-rich aqueous biomass extract was present during enzymatic hydrolysis.

### Example 4: Protein pre-extraction from biomass

Wheat straw was chopped and subjected to pre-extraction with water (10 L per kg straw) at various temperatures (25, 50, 75, 100 °C) and duration (15, 30, 60 min). The mixtures were filtered over a 185 µm filter, resulting in a protein-rich aqueous extract. Wheat straw samples of another batch were subjected to organosolv conditions (190 °C, 60 minutes, solvent = ethanol/water (60/40% wt/wt, 10 L/kg dry matter), 30 mM H₂SO₄, and subsequently to enzymatic hydrolysis (see Example 1, 1.5 g of pulp, 10 FPU Accellerase 1500 per g substrate and 34.9 ml protein extract). It was found that pre-extraction temperatures between 25 and 50°C and durations of 30 and 60 min result in about equal enzymatic hydrolysis conversion rates above 36 h hydrolysis (± 2%), while 75°/30 min and 100°C/15 min result in somewhat lower hydrolysis rates (minus 7-10%).

## Claims

1. A process for enzymatic hydrolysis of cellulose, comprising:
(a) extracting biomass containing both protein and cellulose with an aqueous liquid to produce an extracted biomass and an aqueous extract having a protein content of at least 0.01 g per litre water;
(b) mechanically, chemically and/or thermally pretreating cellulose-containing biomass; and
(c) contacting biomass pretreated in step (b) with:
(i) an enzyme capable of hydrolysing cellulose; and
(ii) the aqueous extract obtained in step (a), optionally after concentration.

2. The process according to claim 1, wherein the extracted biomass obtained in step (a) is used as the cellulose-containing biomass in step (b).

3. The process according to claim 2, wherein the extracted biomass obtained in step (a) is subjected prior to step (b) to extraction with an organic liquid comprising at least 20 wt% of an organic solvent.

4. The process according to any one of claims 1-3, wherein in step (a) the biomass is extracted with water having a temperature of 10-75°C.

5. The process according to claim 4, wherein in step (a) the biomass is extracted with water having a temperature of 15-60°C.

6. The process according to any one of claims 1-5, wherein in step (a) the biomass is extracted with 2-12 l of water per kg of biomass.

7. The process according to any one of claims 1-6, wherein the aqueous extract obtained in step (a) has a protein content of 0.01-50 g/l.

8. The process according to claim 7, wherein the aqueous extract obtained in step (a) has a protein content of 0.1-20 g/l.

9. The process according to any one of claims 1-8, wherein an amount of aqueous extract is used in step (c) which contains between 0.001 and 0.1 g of protein per g pretreated biomass on dry weight basis.

10. The process according to any one of claims 1-9, wherein step (b) comprises heating at a temperature above 75 °C.

11. The process according to claim 10, wherein step (b) comprises heating at a temperature above 120 °C and up to 240°C.

12. The process according to any one of claims 1-11, wherein step (b) comprises treating with a water-miscible organic solvent, optionally in the presence of an acid.

13. The process according to claim 12, wherein the water-miscible organic solvent is selected from lower alcohols and lower ketones.

14. The process according to any one of claims 1-11, wherein step (b) comprises a steam explosion step or an aquathermolysis step.

15. The process according to any one of claims 1-14, wherein in step (c) the enzyme capable of hydrolysing cellulose comprises a mixture of cellulases.

## Patentansprüche

1. Ein Verfahren zur enzymatischen Hydrolyse von Cellulose, umfassend:
(a) Extrahieren von Biomasse, die Protein und Cellulose enthält, mit einer wässrigen Flüssigkeit, um eine extrahierte Biomasse und ein wässriges Extrakt mit einem Gehalt an Protein von wenigstens 0,01 g pro Liter Wasser zu erzeugen;
(b) mechanisches, chemisches und/oder thermisches Vorbehandeln von Celluloseenthaltender Biomasse; und
(c) Kontaktieren der in Schritt (b) vorbehandelten Biomasse mit:
(i) einem Enzym, das in der Lage ist, Cellulose zu hydrolisieren; und
(ii) dem in Schritt (a) erhaltenen wässerigen Extrakt, wahlweise nach Konzentration.

2. Das Verfahren nach Anspruch 1, wobei die in Schritt (a) erhaltene extrahierte Biomasse als die Cellulose-enthaltende Biomasse in Schritt (b) verwendet wird.

3. Das Verfahren nach Anspruch 2, wobei die in Schritt (a) erhaltene extrahierte Biomasse vor dem Schritt (b) einer Extraktion mit einer organischen Flüssigkeit unterworfen wird, die wenigstens 20 Gew.-% eines organischen Lösungsmittels enthält.

4. Das Verfahren nach einem der Ansprüche 1-3, wobei im Schritt (a) die Biomasse mit Wasser extrahiert wird, das eine Temperatur von 10-75 °C aufweist.

5. Das Verfahren nach Anspruch 4, wobei im Schritt (a) die Biomasse mit Wasser extrahiert wird, das eine Temperatur von 15-60 °C aufweist.

6. Das Verfahren nach einem der Ansprüche 1-5, wobei im Schritt (a) die Biomasse mit 2-12 I Wasser pro kg an Biomasse extrahiert wird.

7. Das Verfahren nach einem der Ansprüche 1-6, wobei das im Schritt (a) erhaltene wässerige Extrakt einen Gehalt an Protein von 0,01-50 g/l aufweist.

8. Das Verfahren nach Anspruch 7, wobei das im Schritt (a) erhaltene wässerige Extrakt einen Gehalt an Protein von 0,1-20 g/l aufweist.

9. Das Verfahren nach einem der Ansprüche 1-8, wobei eine Menge an wässerigem Extrakt im Schritt (c) verwendet wird, die zwischen 0,001 und 0,1 g an Protein pro g vorbehandelte Biomasse auf Trockengewichtsbasis enthält.

10. Das Verfahren nach einem der Ansprüche 1-9, wobei der Schritt (b) das Erhitzen bei einer Temperatur oberhalb von 75 °C umfasst.

11. Das Verfahren nach Anspruch 10, wobei der Schritt (b) das Erhitzen bei einer Temperatur oberhalb von 120 °C und bis zu 240 °C umfasst.

12. Das Verfahren nach einem der Ansprüche 1-11, wobei der Schritt (b) das Behandeln mit einem Wasser-mischbaren organischen Lösungsmittel umfasst, wahlweise in Gegenwart einer Säure.

13. Das Verfahren nach Anspruch 12, wobei das Wasser-mischbare organische Lösungsmittel ausgewählt ist aus niederen Alkoholen und niederen Ketonen.

14. Das Verfahren nach einem der Ansprüche 1-11, wobei der Schritt (b) einen Dampfexplosionsschritt oder einen Aquathermolyseschritt umfasst.

15. Das Verfahren nach einem der Ansprüche 1-14, wobei im Schritt (c) das Enzym, das in der Lage ist, Cellulose zu hydrolysieren, eine Mischung von Cellulasen umfasst.

## Revendications

1. Procédé d'hydrolyse enzymatique de cellulose, comprenant :
(a) une extraction de biomasse contenant à la fois des protéines et de la cellulose avec un liquide aqueux pour produire une biomasse extraite et un extrait aqueux ayant une teneur en protéines d'au moins 0,01 g par litre d'eau ;
(b) un prétraitement mécanique, chimique et/ou thermique d'une biomasse contenant de la cellulose ; et
(c) une mise en contact de la biomasse prétraitée à l'étape (b) avec :
(i) une enzyme capable d'hydrolyser la cellulose ; et
(ii) l'extrait aqueux obtenu à l'étape (a), éventuellement après concentration.

2. Procédé selon la revendication 1, dans lequel la biomasse extraite obtenue à l'étape (a) est utilisée en tant que biomasse contenant de la cellulose à l'étape (b).

3. Procédé selon la revendication 2, dans lequel la biomasse extraite obtenue à l'étape (a) est soumise avant l'étape (b) à une extraction avec un liquide organique comprenant au moins 20 % en poids d'un solvant organique.

4. Procédé selon l'une quelconque des revendications 1 à 3, dans lequel, à l'étape (a), la biomasse est extraite avec de l'eau ayant une température de 10 à 75°C.

5. Procédé selon la revendication 4, dans lequel, à l'étape (a), la biomasse est extraite avec de l'eau ayant une température de 15 à 60°C.

6. Procédé selon l'une quelconque des revendications 1 à 5, dans lequel, à l'étape (a), la biomasse est extraite avec 2 à 12 l d'eau par kg de biomasse.

7. Procédé selon l'une quelconque des revendications 1 à 6, dans lequel l'extrait aqueux obtenu à l'étape (a) a une teneur en protéines de 0,01 à 50 g/l.

8. Procédé selon la revendication 7, dans lequel l'extrait aqueux obtenu à l'étape (a) a une teneur en protéines comprise de 0,1 à 20 g/l.

9. Procédé selon l'une quelconque des revendications 1 à 8, dans lequel une quantité d'extrait aqueux est utilisée à l'étape (c), contenant entre 0,0001 et 0,1 g de protéines par g de biomasse prétraitée sur la base du poids à sec.

10. Procédé selon l'une quelconque des revendications 1 à 9, dans lequel l'étape (b) comprend un chauffage à une température supérieure à 75°C.

11. Procédé selon la revendication 10, dans lequel l'étape (b) comprend un chauffage à une température supérieure à 120°C et jusqu'à 240°C.

12. Procédé selon l'une quelconque des revendications 1 à 11, dans lequel l'étape (b) comprend un traitement par un solvant organique miscible dans l'eau, éventuellement en présence d'un acide.

13. Procédé selon la revendication 12, dans lequel le solvant organique miscible dans l'eau est choisi parmi les alcools à chaîne courte et les cétones à chaîne courte.

14. Procédé selon l'une quelconque des revendications 1 à 11, dans lequel l'étape (b) comprend une étape d'explosion de vapeur ou une étape aquathermolytique.

15. Procédé selon l'une quelconque des revendications 1 à 14, dans lequel, à l'étape (c), l'enzyme capable d'hydrolyser la cellulose comprend un mélange de cellulases.
